# EUROPEAN PATENT SPECIFICATION

(11) **EP 1 087 995 B1**
(45) Date of publication and mention of the grant of the patent: **08.09.2004**
(21) Application number: 99931766.2
(22) Date of filing: 23.06.1999
(51) Int. Cl.: C07K 14/49, C07K 14/475, C12N 5/06, C12N 13/00, C12P 21/02

(54) **A METHOD FOR INDUCING PRODUCTION OF GROWTH FACTORS**
VERFAHREN ZUR INDUKTION DER HERSTELLUNG VON WACHSTUMSFAKTOREN
TECHNIQUE DECLENCHANT LA PRODUCTION DE FACTEURS DE CROISSANCE

(30) Priority: 23.06.1998 JP 17574198
(43) Date of publication of application: 04.04.2001
(73) Proprietor: Exogen, Inc., Memphis, Tennessee 38116 (US)
(72) Inventor: ITO, Masayo Teijin Limited, Hino-shi Tokyo 191-0065 (JP); AZUMA, Yoshiaki Teijin Limited, Hino-shi Tokyo 191-0065 (JP); OHTA, Tomohiro Teijin Limited, Hino-shi Tokyo 191-0065 (JP)
(74) Representative: Notaro, Giancarlo
(86) International application number: PCT/US1999/012815
(87) International publication number: WO 1999/067289

(56) References cited:
- US-A- 5 656 450
- WANG ET AL.: "Anabolic effects of 1,25-dihydroxyvitamin D3 on osteoblasts are enhanced by vascular endothelial growth factor produced by osteoblasts and by growth factors produced by endothelial cells" ENDOCRINOLOGY, vol. 138, no. 7, July 1997 (1997-07), pages 2953-2962, XP002118701
- FUNAYAMA H ET AL: "Human monocyte- endothelial cell interaction induces platelet-derived growth factor expression." CARDIOVASCULAR RESEARCH , vol. 37, no. 1, January 1998 (1998-01), pages 216-224, XP002118702
- ZANG ET AL.: "Production and secretion of platelet-derived growth factor AB by cultured human keratinocytes: regulatory effects of phorbol 12-myristate 13-acetate, etretinate, 1,25-dihydroxyvitamin D3, and several cytokines" JOURNAL OF DERMATOLOGY, vol. 22, no. 5, May 1995 (1995-05), pages 305-309, XP002118703 cited in the application

## Description

### FIELD OF THE INVENTION

This invention concerns a method for enhancing production of growth factor in coculture systems. More specifically the invention relates to a method of production of growth factor, including platelet derived growth factor (hereinafter referred to as PDGF).

### BACKGROUND OF THE INVENTION

Concerning methods for enhancing production of growth factors, it was reported that PDGF production was induced by adding active vitamin D to the culture medium of human keratinocytes by Zang et al., *J Dermatol* 22, 305-309, (1995) in a single culture system. Methods for enhancing production of PDGF in coculture systems are not known at present. And a more efficient method for enhancing production of growth factors is needed.

In view of the above, a series of assiduous studies were conducted to provide a more efficient method for enhancing production of growth factors in coculture systems.

As an example of these studies, Wang et al (Endocrinology, vol. 138. no. 7, July 1997, pages 2953-2962 describe the effects of 1,25-dihydroxyvitamin D₃ on a coculture of osteoblast (HOB) and endothelial (HUVEC) cells; in the case of co-culturing HOB and HUVEC Wang demonstrates that the active form of vitamin D, i.e. 1,25-dihydroxyvitamin D₃, performs the stimulation of a vascular endothelial growth factor receptor gene expression on the endothelial cells followed by increased production of osteotropic growth factors.

The US patent 5 656 450 describes a method to convert a latent transforming growth factor beta (TGFβ) already produced by the cells in an active form by means of ultrasound.

### SUMMARY OF THE INVENTION

This invention provides embodiments comprising methods for enhancing production of growth factor in a coculture system by (1) adding an active form of vitamin D to coculture medium, (2) exposing the cells in a coculture medium to ultrasound and (3) adding an active form of vitamin D to a coculture medium and exposing the cells in the cocultun medium to ultrasound. The invention is described below in detail.

### BRIEF DESCRIPTION OF THE DRAWING

The Figure shows the amounts of PDGF-AB in the coculture medium
wherein:
a) Control group
b) Ultrasound exposure group
c) Active vitamin D treatment group
d) Ultrasound exposure + active vitamin D treatment group

The asterisk (*) and the double asterisk (**) indicate a P value of <0.05 and <0.01, respectively, compared with the control group according to the results of the Dunnett's multiple comparison test.

### DETAILED DESCRIPTION OF THE INVENTION

The invention is applicable to growth factors such as basic fibroblast growth factor (bFGF), transforming growth factor-ß (TGF-ß), and PDGF. Among them, PDGF is preferred.

These growth factors have been suggested to play important roles in the tissue repair process, e.g., in fracture repair [Joyce et al. *Prog. Clin. Biol. Res.* 365, 391-416, (1991)]. One of them, PDGF has been found to participate in extremely important vital phenomena in body development, cell differentiation, cell proliferation, etc. In addition, PDGF possesses growth stimulating activity toward almost all mesodermally derived cells. Because PDGF possesses such activities, it has been expected to be used as a therapeutic agent, such as a neovascularization promoting agent or a wound healing agent. Actually, it has been found to play an important role in wound healing process [H. Hosgood, *Vet Surg* 22, 490-495 (1993), United States Patent No. 5,457,093]. In addition, fracture repair can be accelerated by its local injection, and it can be used as a therapeutic agent for fracture.

Coculture systems of the invention are the culture of two or more kinds of cells which produce growth factors, in the same medium. Among them, the combination of osteoblasts and endothelial cells is preferred. Osteoblasts as described herein mean human osteosarcoma cell (SaOS-2), HOS, M063, etc. These cells can be obtained from the American Type Culture Collection, and experimentally used by culturing, and subculturing as described below.

Endothelial cells as described herein means human umbilical vein endothelial cells (RUVEC), microvascular endothelial cells, pulmonary endothelial cells, etc. These cells can be obtained from Sanko Junyaku Co. Ltd., Takara Co. Ltd., and Iwaki Glass Co. Ltd., of Japan etc. These cells can be used by culturing, and subculturing in a medium for endothelial cells. The coculture system of osteoblasts and endothelial cells is established by culturing osteoblasts in the culture medium as described below, and adding endothelial cells onto osteoblasts the day after resulting in a coculture system.

Culture media used in the invention for the coculture system mean McCoy's 5A modified medium, a-MEM containing 5-15% fetal calf serum, etc. Among them, McCoy's 5A modified medium containing 10 % fetal calf serum is preferred.

The active form of vitamin D used in the invention means a form of vitamin D that possesses physiological activities, such as calcium and bone metabolism regulating activities, as distinguished from forms of vitamin D that have no physiological activity, and includes active forms of vitamin D₂, active forms of vitamin D₃, and their derivatives. Actual examples include 1-hydroxyvitamin D, 1,24-dihydroxyvitamin D, 1,25-dihydroxyvitamin D, 1,24,25-trihydroxyvitamin D, 24,24-difluoro-1,25-dihydroxyvitamin D, and 26,26,26,27,27,27-hexafluoro-1,25-dihydroxyvitamin D. Among them, 1-hydroxyvitamin D₃, 1,24(R)-dihydroxyvitamin D₃, and 1,25-dihydroxyvitamin D₃ are preferred. Concentrations which can be added to the culture medium are 1x10⁻¹⁰M to 1x10⁻⁷M, and the range of concentration of 1x10⁻⁵M to 3x10⁻⁸M is preferable.

In a preferred embodiment low-intensity ultrasound is that which is not associated with thermal events. Low-intensity ultrasound consists of a frequency of 1.3 to 2 MHz, a repeat cycle of 100 to 1,000 Hz, a burst width of 10 to 2000 µs, and an intensity up to 100 mW/cm². It is further contemplated that other ultrasound energy can be used either with or without thermal effect.

Exposing the undersurface of the wells containing the coculture to ultrasound using an Exogen Co. ultrasound output for 20 minutes a day for 4 consecutive days is preferable.

The characteristics of the ultrasound output employed below were a pulse burst of 200 µs, a frequency of 1.5 MHz, a 1kHz repeat cycle, and the intensity was 30mW/cm².

The ultrasound machine used in these experiments is described in United States Patent (No. 4,530,360) as a basic non-invasive treatment technique and device for applying ultrasound pulses transcutaneously from outside the patient's body close to the affected site. Low-intensity ultrasound consists of a frequency of 1.3 to 2 MHz, a repeat cycle of 100 to 1,000 Hz, a burst width of 10 to 2000 µs, and an intensity up to 100mW/cm². The preferred duration of ultrasound exposure is up to 20 minutes.

In U.S. Patent No. 5,520,612 to Winder et al. preferred ultrasound treatment systems are also disclosed. In those systems low intensity ultrasound at a frequency range of 20 kHz to 10 MHz are employed. The repeat cycle range is 5 to 10 kHz. The duty cycle is from about 5 to 90% and the intensity is up to 100mW/cm².

In another United States patent (USP 5,003,965), Talish et al. have described an ultrasound treatment system that consists of a body-applicator-unit connected to a remote control unit by a covered optical cable, and low-intensity ultrasound in this system consists of a frequency of 1.3 to 2 MHz, and an intensity up to 1 to 50mW/cm².

The method for enhancing production of growth factors in the coculture system in this invention involves the combination of adding active vitamin D to a coculture medium containing cells producing growth factor, and also to exposing cells to ultrasound is preferable.

### EXAMPLES

The following specific examples are given to illustrate the present invention.

### Examples 1 to 3 and Comparative Example

A 2-mL volume of human osteoblastic cells (SaOS-2) was seeded at a density of 2x10⁵ cells/well into a 6-well plate coated with human type 1 collagen. SaOS-2 is an osteoblastic cell line that was established in 1975 from a Caucasian female osteosarcoma, and it was obtained from the American Type Culture Collection. McCoy's 5A medium to which 10% fetal calf serum (FCS) had been added (10% FCS - McCoy's 5A) was used as the culture medium. The next day, 0.5 mL of human umbilical vein endothelial cells (HUVECs) was added to each well at a density of 3x10⁵ cells/well. The HUVECs were obtained from Sanko Junyaku Co. Ltd.

Each group is shown as below.

Example 1; The undersurface of the wells was exposed to ultrasound for 20 min a day for 4 consecutive days.

Example 2; 10⁻⁸M 1,25-dihydroxyvitamin D₃ was added to the culture medium.

Example 3; 10⁻⁸M 1,25-dihydroxyvitamin D₃ was added to the culture medium and the undersurface of the wells was exposed to ultrasound for 20 min a day for 4 consecutive days.

In the Comparative Example and Example 1, concerning active vitamin D treatment, the culture medium was replaced with 10% FCS - McCoy's 5A containing 0.01 % ethanol (vehicle for 1,25-dihydroxyvitamin D3). The medium was changed 2 days later.

In Example 2 and Example 3, the culture medium was replaced with 10% FCS - McCoy's 5A containing 1x10⁻⁸M 1,25-dihydroxyvitamin D3. The medium was changed 2 days later.

Concerning ultrasound exposure, in Comparative Example and Example 2, there was no exposure to ultrasound. In Example I and Example 3, the exposure to low-intensity ultrasound with an Exogen Co. ultrasound output unit, 20 minutes a day, for 4 consecutive days. The characteristics of the ultrasound output were a pulse width of 200 µs, a frequency of 1.5 MHz, a 1kHz repeat cycle, and the intensity was 30mW/cm².

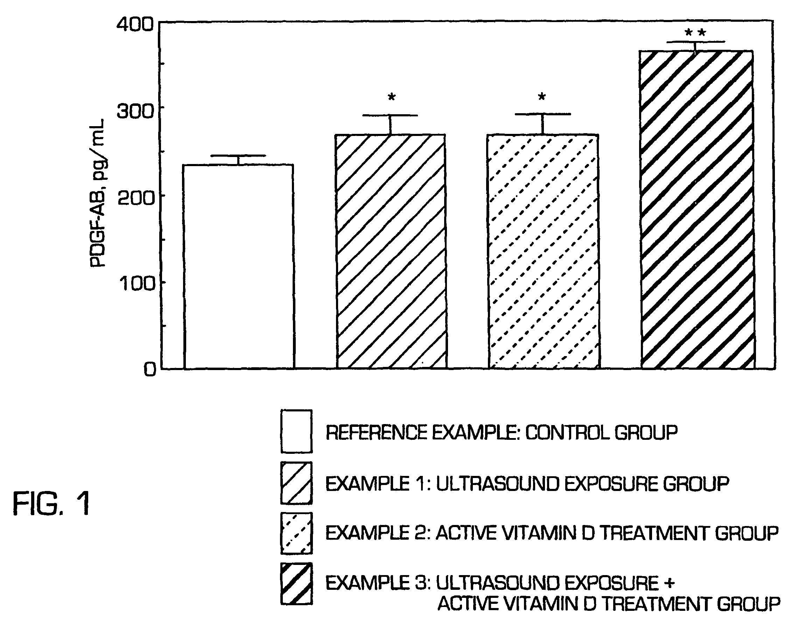

The culture medium was collected the following day, and the concentration of PDGF-AB in the culture medium was measured with an Amersham Co. ELISA kit that specifically measures PDGF-AB. The results obtained are shown in Figure 1.

As clearly shown in Figure 1, the amount of produced PDGF-AB was significantly increased in Example 1, 2 and 3 compared with the Comparative Example. Furthermore, a greater increase in PDGF-AB in Example 3 was observed among the Example groups.

## Claims

1. A method for enhancing production of a flatelet derived growth factor comprising the step of: coculturing cells producing a flatelet derived growth factor in the presence of active vitamin D.

2. A method for enhancing production of a a flatelet derived growth factor comprising the step of coculturing cells producing a flatelet derived growth factor while exposing the cells to ultrasound.

3. A method for enhancing production of a flatelet derived growth factor according to claim 1, comprising the step of exposing the cells to ultrasound.

4. A method of claim 1, claim 2 or claim 3 wherein the coculturing is of osteoblasts and vascular endothelial cells.

## Patentansprüche

1. Ein Verfahren zum Steigern der Produktion eines Thrombozytenwachstumsfaktors, das den Schritt des Kokultivierens von einen Thrombozytenwachstumsfaktor produzierenden Zellen, in Anwesenheit von aktivem Vitamin D, beinhaltet.

2. Verfahren zum Steigern der Produktion eines Thrombozytenwachstumsfaktors, das den Schritt des Kokultivierens von einen Thrombozytenwachstumsfaktor produzierenden Zellen, während die Zellen Ultraschall ausgesetzt werden, beinhaltet.

3. Verfahren zum Steigern der Produktion eines Thrombozytenwachstumsfaktors gemäß Anspruch 1, das den Schritt beinhaltet, die Zellen Ultraschall auszusetzen.

4. Verfahren gemäß Anspruch 1, Anspruch 2 oder Anspruch 3, wobei Osteoblasten und vaskuläre Endothelzellen kokultiviert werden.

## Revendications

1. Un procédé destiné à accroître la production d'un facteur de croissance dérivé des plaquettes comportant l'étape de : co-cultiver des cellules produisant un facteur de croissance dérivé des plaquettes en présence de vitamine D active.

2. Un procédé destiné à accroître la production d'un facteur de croissance dérivé des plaquettes comportant l'étape de co-cultiver des cellules produisant un facteur de croissance dérivé des plaquettes tout en exposant les cellules à des ultrasons.

3. Un procédé destiné à accroître la production d'un facteur de croissance dérivé des plaquettes selon la revendication 1, comportant l'étape d'exposer les cellules à des ultrasons.

4. Un procédé de la revendication 1, la revendication 2 ou la revendication 3 dans lequel des ostéoblastes et des cellules endothéliales vasculaires sont co-cultivés.
